# EUROPEAN PATENT APPLICATION

(11) **EP 4 801 234 A1**
(43) Date of publication of application: **02.09.2026**
(21) Application number: 24882484.9
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT, PHOTOELECTRIC CONVERSION DEVICE, AND METHOD FOR MANUFACTURING PHOTOELECTRIC CONVERSION ELEMENT**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 09.10.2024 JP 2024177318; 23.10.2024 JP 2024186701
(71) Applicant: Canon Kabushiki Kaisha, Tokyo 146-8501 (JP)
(72) Inventor: TANAKA, Kentaro, Tokyo 146-8501 (JP); NISHIDA, Tsutomu, Tokyo 146-8501 (JP); NAKAMURA, Nobuhiro, Tokyo 146-8501 (JP); NASUNO, Yuki, Tokyo 146-8501 (JP); OHSAWA, Tatsuya, Tokyo 146-8501 (JP); MAKISUMI, Kohei, Tokyo 146-8501 (JP); MASUDA, Yuki, Tokyo 146-8501 (JP)
(74) Representative: TBK
(86) International application number: PCT/JP2024/038062
(87) International publication number: WO 2025/089376

(57) **Abstract**

Provided is a composition improved in dispersion stability and in photoelectric conversion efficiency of a photoelectric conversion element using the composition.

The photoelectric conversion element includes: a first electrode; a second electrode; and a photoelectric conversion layer arranged between the first electrode and the second electrode, the photoelectric conversion layer containing a crystal having a perovskite structure. The photoelectric conversion element further includes a charge-transporting layer that is arranged between the photoelectric conversion layer and the first electrode, and is brought into contact with the first electrode. The charge-transporting layer contains a particle of a cyclic conjugated compound in which pyrrole rings are bonded by conjugated bonds, a calixarene compound, and an insulating resin. The first electrode is a carbon electrode.

## Description

### [Technical Field]

The present invention relates to a photoelectric conversion element, a photoelectric conversion apparatus, and a method of producing a photoelectric conversion element.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Currently, an n-p diode-type silicon (Si) single crystal-based solar cell having a light energy conversion efficiency of more than 20% is widely known, and is actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate, and hence a cost reduction can be expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion characteristic. For example, in Patent Literature 1, there is a description of a technology for making it difficult for peeling between a hole-transporting layer and an electrode to occur by incorporating an organic semiconductor and an insulating polymer compound having a glass transition point of 100°C or more into the hole-transporting layer. In addition, in Patent Literature 2, there is a description of a technology for suppressing a reduction in conversion efficiency during continuous use for a long time by incorporating a phthalocyanine compound and an aromatic ring compound having a hydroxy group, which is different from the phthalocyanine compound, into a charge-transporting layer.

### [Citation List]

### [Patent Literature]

PTL 1: Japanese Patent Laid-Open No. 2018-170382
PTL 2: Japanese Patent Laid-Open No. 2024-60579

### [Summary of Invention]

### [Technical Problem]

According to investigations made by the inventors of the present invention, it has been found that, in the photoelectric conversion element described in each of Patent Literatures 1 and 2, there is room for improvement in photoelectric conversion efficiency when a carbon electrode is used.

Accordingly, the present invention is directed to providing a photoelectric conversion element improved in photoelectric conversion efficiency when a carbon electrode is used.

### [Solution to Problem]

The above-mentioned provision is achieved by the present invention described below. That is,
the present invention is directed to a photoelectric conversion element including:
a first electrode;
a second electrode; and
a photoelectric conversion layer arranged between the first electrode and the second electrode, the photoelectric conversion layer containing a crystal having a perovskite structure,
wherein the photoelectric conversion element further includes a charge-transporting layer that is arranged between the photoelectric conversion layer and the first electrode, and is brought into contact with the first electrode,
wherein the charge-transporting layer contains a particle of a cyclic conjugated compound in which pyrrole rings are bonded by conjugated bonds, a calixarene compound, and an insulating resin, and
wherein the first electrode is a carbon electrode.

### [Advantageous Effects of Invention]

The present invention can provide the photoelectric conversion element improved in photoelectric conversion efficiency when a carbon electrode is used.

### [Brief Description of Drawings]

[Fig. 1]

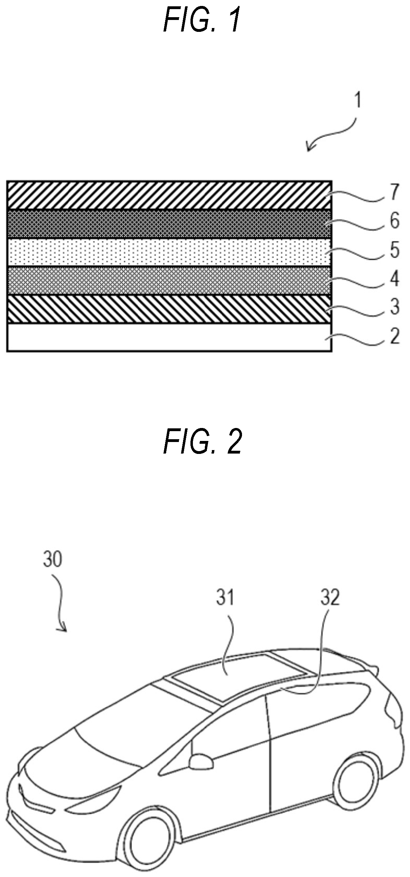

   Fig. 1 is a schematic sectional view in the thickness direction of a photoelectric conversion element according to a first embodiment of the present invention.
[Fig. 2]
   Fig. 2 is a perspective view for schematically illustrating a moving body including the photoelectric conversion element according to one embodiment of the present invention.
[Fig. 3]

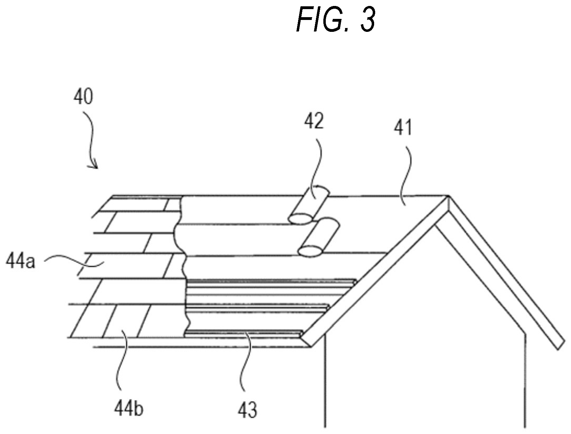

   Fig. 3 is a perspective view for schematically illustrating a building material including the photoelectric conversion element according to one embodiment of the present invention.

### [Description of Embodiments]

A photoelectric conversion element of the present invention is a photoelectric conversion element including: a first electrode; a second electrode; and a photoelectric conversion layer arranged between the first electrode and the second electrode, the photoelectric conversion layer containing a crystal having a perovskite structure. The photoelectric conversion element further includes a charge-transporting layer that is arranged between the photoelectric conversion layer and the first electrode, and is brought into contact with the first electrode. The charge-transporting layer contains a particle of a cyclic conjugated compound in which pyrrole rings are bonded by conjugated bonds, a calixarene compound, and an insulating resin. The first electrode is a carbon electrode.

As a result of investigations, the inventors of the present invention have found that a photoelectric conversion element having excellent conversion efficiency is achieved by the above-mentioned configuration. The reason why the photoelectric conversion element having excellent conversion efficiency can be obtained in the present invention is not clear in detail, but a possible reason therefor is as described below.

When the particle of the cyclic conjugated compound in which the pyrrole rings are covalently bonded is formed into a film as a charge-transporting substance, the charge-transporting layer exhibits a high hole-transporting ability. However, when the layer is combined with a carbon electrode, the exchange of charge with the electrode is insufficient, and photoelectric conversion efficiency sometimes reduces in some cases. In addition, in the related-art investigations made by the inventors of the present invention, the following possibility has been suggested: an electronic interaction with the carbon electrode is increased and the series resistance of the film is reduced by the addition of a calixarene compound. However, it has been found that the number of the molecules of the calixarene compound arranged in the vicinity of the electrode is not sufficient, and an increase in photoelectric conversion efficiency is limited.

Meanwhile, the following has been inferred: when the calixarene compound and the insulating resin are added, the calixarene compound is appropriately arranged in the charge-transporting layer by dispersing the calixarene compound in a molecular state through an interaction with the resin while allowing the resin to secure leak resistance; thus, a sufficient interaction with the carbon electrode can be achieved, and hence the interaction contributes to an improvement in conversion efficiency.

In the present invention, the particle of the cyclic conjugated compound in which the pyrrole rings are bonded by conjugated bonds, the particle being incorporated into the charge-transporting layer, is more preferably a particle of a phthalocyanine compound. The phthalocyanine compound may have a central element, and examples of the central element include Ga, Cu, Ti, Zn, Si, V, Pb, Pt, Co, Sn, Mg, Fe, Al, and Mn. Of those, a metal phthalocyanine compound having a metal element in its center is preferred. Of those, a gallium phthalocyanine compound in which the central element is Ga or a titanyl phthalocyanine compound in which the central metal is Ti is preferred. A hydroxygallium phthalocyanine compound is more preferred.

The charge-transporting particle can more efficiently transport charge generated in the photoelectric conversion layer.

Specific examples of the insulating resin incorporated into the charge-transporting layer in the present invention include a polyacetal resin, an acrylic resin, a polyarylate resin, a polycarbonate resin, a polyvinyl acetate resin, a polyester resin, a polyamide resin, a polyurethane resin, and a polystyrene resin. In addition, the glass transition temperature of the insulating resin is preferably 95°C or less. When the glass transition temperature falls within the range, the insulating resin is easily brought into close contact with a charge-transporting material, and hence a more effective charge distribution can be formed. The glass transition temperature may be determined with a differential scanning calorimeter (DSC).

In the present invention, the insulating resin is preferably a polyvinyl acetal resin or a polyvinyl butyral resin. The insulating resin is easily brought into close contact with the charge-transporting material (the particle of the cyclic conjugated compound in which the pyrrole rings are bonded by conjugated bonds), and hence a more effective charge distribution can be formed.

In the present invention, the calixarene compound incorporated into the charge-transporting layer is specifically represented by the following formula [A]: 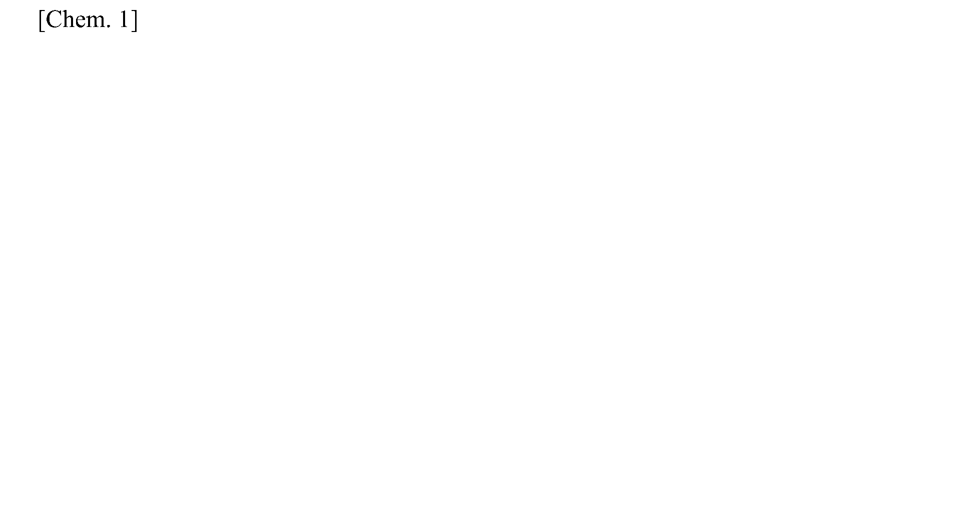 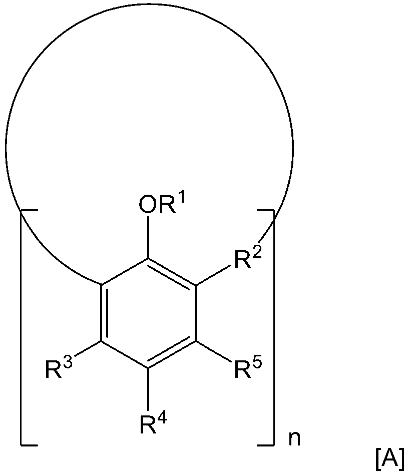 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, -Y- of the -Y-Ar group represents -CH=N-, -CH=CH-, or -N=N-, and Ar thereof represents a substituted or unsubstituted aromatic hydrocarbon group, or a substituted or unsubstituted heterocyclic group, and "n" represents an integer of 3 to 20.

Examples of the alkyl group represented by each of R¹ to R⁵ described above include a methyl group, an ethyl group, a propyl group, and a butyl group.

Examples of the aromatic hydrocarbon group represented by each of R³ to R⁵ include benzene, naphthalene, fluorene, phenanthrene, anthracene, fluoranthene, and pyrene.

In addition, examples of the heterocyclic group represented by each of R³ to R⁵ include furan, thiophene, pyridine, indole, benzothiazole, carbazole, benzocarbazole, acridone, dibenzothiophene, benzoxazole, benzotriazole, oxathiazole, thiazole, phenazine, cinnoline, and benzocinnoline.

In addition, examples of a substituent that the alkyl group, the phenylazo group, the aromatic hydrocarbon group, or the heterocyclic group represented by each of R² to R⁵ may have include: alkyl groups, such as a methyl group, an ethyl group, a propyl group, and a butyl group; alkoxy groups, such as a methoxy group and an ethoxy group; dialkylamino groups, such as a dimethylamino group and a diethylamino group; alkoxycarbonyl groups, such as a methoxycarbonyl group and an ethoxycarbonyl group; halogen atoms, such as a fluorine atom, a chlorine atom, and a bromine atom; a hydroxy group; a nitro group; a cyano group; and a halomethyl group.

In the aromatic ring compound having a calixarene structure represented by the formula [A], "n" preferably represents 4 to 8 and its molecular weight is preferably 10,000 or less from the viewpoint of its molecular size in order to easily improve dispersion stability.

In addition, in the present invention, R¹ preferably represents a hydrogen atom, a methyl group, an ethyl group, or a propyl group each independently for "n" repeating units. In addition, R² preferably represents a methylene group, an ethylene group, or a trimethylene group each independently for "n" repeating units. It is preferred that R³ and R⁵ each represent a hydrogen atom, and R⁴ represent a nitrophenylazo group or a dinitrophenylazo group each independently for "n" repeating units.

Of those, specific examples of the calixarene compound to be particularly preferably used in the present invention are given below. In the present invention, the charge-transporting layer preferably contains, as a dispersant, at least one selected from the group consisting of: a compound represented by the following formula [C-1]; a compound represented by the following formula [C-2]; a compound represented by the following formula [C-3]; and a compound represented by the following formula [C-4], and more preferably contains all the four compounds (mixed product).

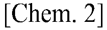

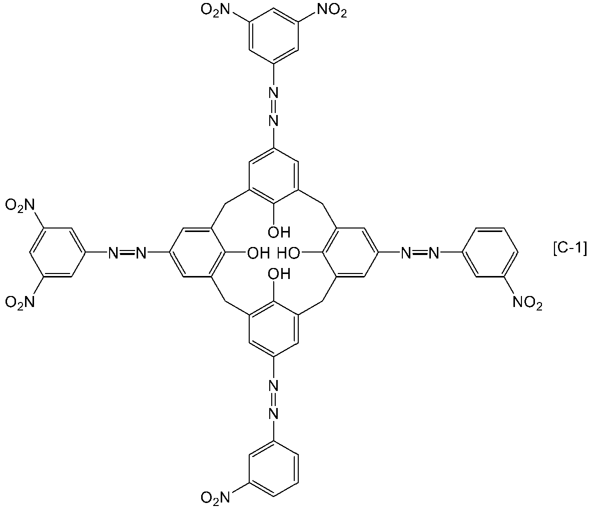

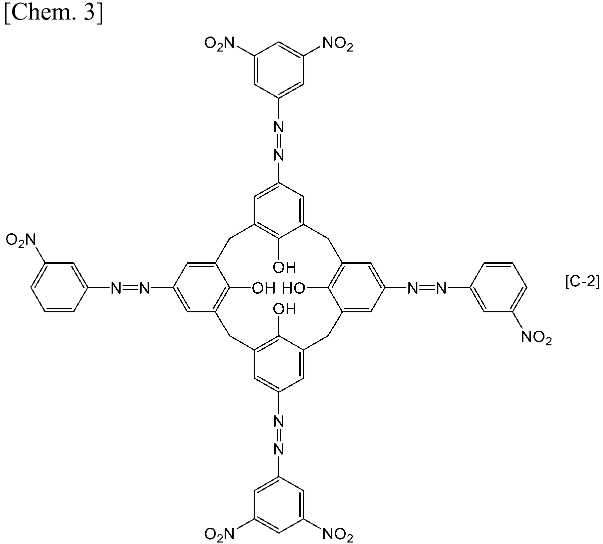

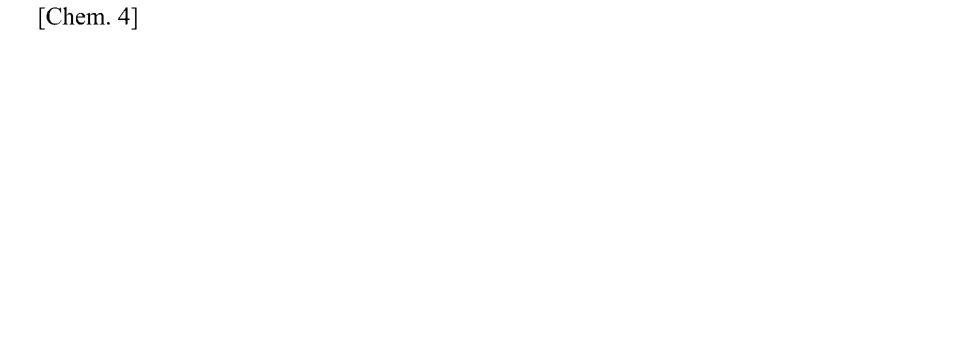

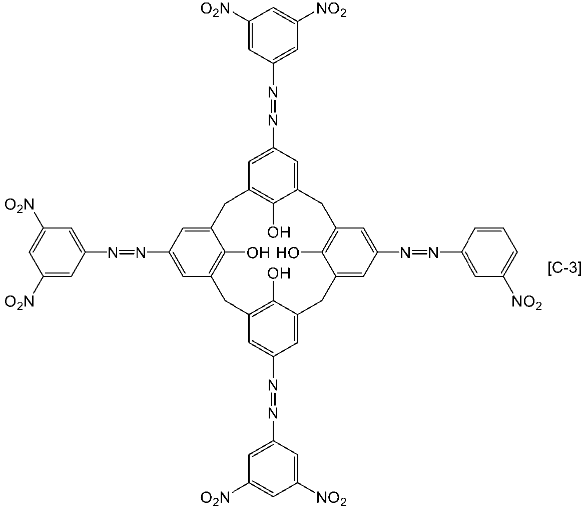

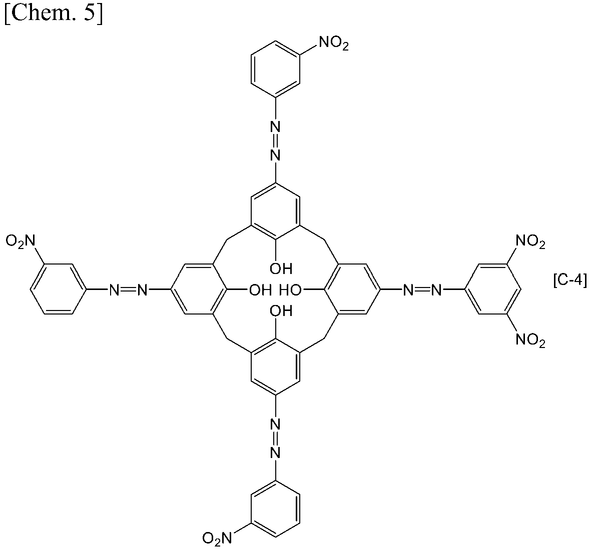

In the present invention, the first electrode is a carbon electrode, and preferably contains at least one of carbon black, graphite, or a carbon nanotube. When the carbon electrode is used, an electrode of a photoelectric conversion element excellent in conversion efficiency is provided by an inexpensive and simple material and production method.

The effects of the present invention can be achieved when the respective configurations synergistically exhibit effects on each other as in the mechanism as described above.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and observing the element distribution of a specific element. The analysis of each layer may be performed by peeling and removing a layer from a completed photoelectric conversion element to expose the layer to be analyzed. In the present invention, for the quantification of a volume ratio, the area ratio of an exposed surface or a cross section is used as the volume ratio of the layer.

Fig. 1 is a sectional view for schematically illustrating the configuration of the photoelectric conversion element according to one embodiment of the present invention. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 with an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes (the second electrode 3 and the first electrode 7), and may be omitted in some cases. A form in which the electron-transporting layers 4 and photoelectric conversion layers 5 are laminated may be adopted. Such form may also be referred to as "tandem structure." The respective members are described below.

### [Photoelectric Conversion Element]

The photoelectric conversion element of the present invention includes: the first electrode; the second electrode; and the photoelectric conversion layer arranged between the first electrode and the second electrode, the photoelectric conversion layer containing the crystal having a perovskite structure, the photoelectric conversion element being characterized in that: the photoelectric conversion element further includes the charge-transporting layer between the photoelectric conversion layer and the first electrode; the charge-transporting layer contains the particle of the cyclic conjugated compound in which the pyrrole rings are bonded by conjugated bonds, the calixarene compound, and the insulating resin; and the first electrode is a carbon electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The present invention relates to a method of producing a photoelectric conversion element including a photoelectric conversion layer, a charge-transporting layer, and an electrode, the method including, in this order, the steps of: forming the photoelectric conversion layer containing a crystal having a perovskite structure; forming the charge-transporting layer containing a particle of a cyclic conjugated compound in which pyrrole rings are bonded by conjugated bonds, a calixarene compound, and an insulating resin; and forming the electrode by applying a coating liquid.

According to the production method of the present invention, when an electrode is produced by using a coating liquid, a photoelectric conversion element improved in photoelectric conversion efficiency can be produced inexpensively and simply.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

The photoelectric conversion element of the present invention includes the first electrode and the second electrode. The first electrode is a carbon electrode, and preferably contains at least one selected from the group consisting of: carbon black; graphite; and a carbon nanotube. A material for the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The electrode may be a patterned electrode.

### [Photoelectric Conversion Layer]

The photoelectric conversion element of the present invention includes the photoelectric conversion layer arranged between the first electrode and the second electrode, the photoelectric conversion layer containing the crystal having a perovskite structure. The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚNₘHₙ ("p", "m", and "n" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4]. "n" represented in each of the following general formulae represents a positive integer.

R'₂Aₙ₋₁BₙX₃ₙ₊₁ [2]

R"Aₙ₋₁BₙX₃ₙ₊₁ [3]

R‴AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule that may have a substituent or a metal. Specifically, ethylammonium, propylammonium, n-butylammonium, n-hexylammonium, n-octylammonium, 1,6-hexanediammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (pF-PEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I_{16,} (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of x1 to x5 may be changed in accordance with purposes. The combinations of x1 to x5 are, for example, as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of the electron in the organic-inorganic perovskite compound increases, and the photoelectric conversion efficiency of the photoelectric conversion element is improved.

In addition, the photoelectric conversion layer according to the present invention may include a material except the crystal having an organic-inorganic perovskite structure to the extent that the photoelectric conversion efficiency and the charge transportability are not impaired.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

The photoelectric conversion element of the present invention further includes the charge-transporting layer arranged between the photoelectric conversion layer and the first electrode, and the charge-transporting layer is formed of the particle of the cyclic conjugated compound in which the pyrrole rings are bonded by conjugated bonds, the calixarene compound, and the insulating resin. The respective items, such as the particle of the cyclic conjugated compound, the calixarene compound, and the insulating resin, are as described above.

In the present invention, the charge-transporting layer contains the particle of the cyclic conjugated compound, which is a P-type semiconductor, and the insulating resin, and the content mass of the particle of the cyclic conjugated compound in the charge-transporting layer is preferably 5 to 30 times with respect to the content mass of the insulating resin in the charge-transporting layer. The insulating resin has a volume resistivity of 10⁸ Ω·cm or more. In addition, the content mass of the particle of the cyclic conjugated compound in the charge-transporting layer is preferably 2 to 20 times with respect to the content mass of the calixarene compound in the charge-transporting layer.

The thickness of the charge-transporting layer is preferably 1 to 1,000 nm, more preferably 5 to 500 nm, particularly preferably 10 to 200 nm.

The charge-transporting layer may be formed by preparing a coating liquid for a charge-transporting layer containing the above-mentioned respective materials and a solvent, forming a coating film of the liquid on the photoelectric conversion layer, and drying the coating film. Examples of the solvent to be used for the coating liquid include an alcohol-based solvent, a ketone-based solvent, an etherbased solvent, an ester-based solvent, and an aromatic hydrocarbon-based solvent. Of those solvents, an alcohol-based solvent or an aromatic hydrocarbon-based solvent is preferred.

### [Electron-transporting Layer]

In the photoelectric conversion element of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide. In particular, tin oxide may be obtained through the reaction of tin(II) chloride, tin(IV) chloride, tin(II) chloride dihydrate, or tin(IV) chloride pentahydrate.

A preferred lower limit of the thickness of the electron-transporting layer 4 is 1 nm, and a preferred upper limit thereof is 2,000 nm. When the thickness of the electron-transporting layer 4 is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### [Control of Particle Diameter of Particle of Cyclic Conjugated Compound]

The particle diameter of the particle of the cyclic conjugated compound may be changed by dispersing the coating liquid for a charge-transporting layer with a paint shaker, and the particle diameter may be reduced by increasing a dispersion time. In addition, the particle diameter may be reduced by further subjecting the coating liquid for a charge-transporting layer to a centrifuge.

### <Application Examples>

Application examples of the present invention are directed to a photoelectric conversion apparatus, a moving body, and a building material.

### [Photoelectric Conversion Apparatus]

A photoelectric conversion apparatus of the present invention includes the above-mentioned photoelectric conversion element. The photoelectric conversion apparatus may be formed by using the photoelectric conversion elements of the present invention. When the photoelectric conversion elements are connected, such photoelectric conversion apparatus may also be referred to as "photoelectric conversion cell" or "photoelectric conversion module." Photoelectric conversion elements having different absorption wavelengths may be laminated as the photoelectric conversion elements to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

A moving body of the present invention includes the above-mentioned photoelectric conversion element. Fig. 2 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

A building material of the present invention includes the above-mentioned photoelectric conversion element. Fig. 3 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

In addition to the application examples described above, the following application examples are given: portable devices, such as a calculator, a sensor, and a small solar panel; wearable devices, such as a glasses-type terminal, a watch-type terminal, and a portable medical device; sheet structures supported by frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### (Example 1)

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) having sealed therein 5 parts of glass beads, followed by filtration and drying to provide a particle 1 (specific gravity: 1.6).

### <Production of Resin Solution 1>

1.0 Grams of polyvinyl butyral (product name: BM-2, manufactured by Sekisui Chemical Co., Ltd., glass transition temperature: 71°C) was dissolved in 19 g of 2-propanol by stirring for 24 hours to provide a resin solution 1.

### [Formation of Electron-transporting Layer]

A 25 mm×25 mm square glass substrate with ITO was washed, and a tin(II) oxide colloidal solution (15% water dispersion, manufactured by Alfa Aesar) diluted fivefold was applied thereonto by spin coating, followed by heating at 150°C for 30 minutes to form a thin film-shaped electron-transporting layer having a thickness of 16 nm.

### [Formation of Photoelectric Conversion Layer]

0.487 Grams of lead bromide, 1.034 g of formamidinium iodide, 2.903 g of lead iodide, and 0.139 g of methylammonium bromide were dissolved in 4.25 g of N,N-dimethylformamide and 1.216 g of dimethyl sulfoxide, and the solution was stirred for 1 hour (solution 1). Further, 0.100 g of cesium iodide was dissolved in 0.285 g of dimethyl sulfoxide, and the solution was stirred for 1 hour (solution 2). After that, the cesium iodide solution (solution 2) was added to the solution 1 to prepare a photoelectric conversion layer coating liquid. The coating liquid was applied onto the electron-transporting layer by spin coating in accordance with a poor solvent method to form a photoelectric conversion layer formed of Cs_{0.05}(FA_{0.83}MA_{0.17})_{0.95}Pb(I_{0.83}Br_{0.17})₃, the layer having a thickness of 600 nm.

### [Formation of Charge-transporting Layer]

0.1 Grams of the particle 1 and 0.01 g of a mixture of compounds represented by the respective formulae [C-1] to [C-4] serving as calixarene compounds were mixed with 10.6 g of 2-propanol. The mass ratio "[C-1]:[C-2]:[C-3]:[C-4]" was set to 1:1:1:1. 11 Grams of beads (zirconia beads, Torayceram (trademark) zirconia beads, 0.3 mm) were loaded into the mixture, followed by dispersion with a paint shaker (manufactured by Toyo Seiki Co., Ltd.) for 7 hours. After that, 0.2 g of the resin solution 1 was added thereto, and the mixture was dispersed with the paint shaker again for 6 hours to prepare a charge-transporting layer solution 1. The charge-transporting layer solution 1 was applied onto the photoelectric conversion layer by spin coating to form a charge-transporting layer having a thickness of 160 nm.

### [Formation of First Electrode]

A carbon paste (product name: JELCON CH-8, manufactured by Jujo Chemical Co., Ltd.) was applied onto the charge-transporting layer, and then heated at 120°C for 15 minutes to form electrodes each having an area of 0.09 cm² at 10 locations. Thus, a photoelectric conversion element was obtained.

### [Analysis of Compound Amount]

The electrode surface of the photoelectric conversion element was peeled off so that the surface of the charge-transporting layer was exposed. The surface of the charge-transporting layer was wiped with a cotton swab or the like with a solvent, dissolved in deuterated sulfuric acid, and subjected to ¹H-NMR measurement (apparatus: AVANCE III 500 manufactured by BRUKER). In addition, the presence of a compound was determined by subjecting the peeled-off charge-transporting layer components to mass and structure analyses through GPC, MALDI-TOF-MS, IR, gas chromatography, or elemental analysis, such as XPS or EDX.

In addition, the thickness of the photoelectric conversion element was determined with a cross-sectional SEM (apparatus: SmartSEM manufactured by Carl Zeiss Co., Ltd.) after the cutting of the photoelectric conversion element and the fixing of the sample to a tilted sample stage.

### (Example 2)

A photoelectric conversion element is obtained in the same manner as in Example 1 except that the polyvinyl butyral is changed to polyvinyl butyral (product name: BL-5Z, manufactured by Sekisui Chemical Co., Ltd., glass transition temperature: 67°C).

### (Example 3)

A thin film layer having a thickness of 20 nm is formed between the charge-transporting layer and the photoelectric conversion layer by: dissolving 2.49 mg of 2-phenylethylamine hydroiodide into 1 mL of 2-propanol to prepare a solution for a thin film layer; and applying the solution for a thin film layer onto the photoelectric conversion layer by spin coating. A photoelectric conversion element is obtained in the same manner as in Example 1 except the foregoing.

### (Example 4)

A photoelectric conversion element is obtained in the same manner as in Example 1 except that the particle 1 serving as the particle of the cyclic conjugated compound in which the pyrrole rings are bonded by conjugated bonds is changed to a chlorogallium phthalocyanine (ClGaPc) particle.

### (Example 5)

A photoelectric conversion element is obtained in the same manner as in Example 1 except that the particle 1 serving as the particle of the cyclic conjugated compound in which the pyrrole rings are bonded by conjugated bonds is changed to a copper phthalocyanine (CuPc) particle.

### (Example 6)

A photoelectric conversion element is obtained in the same manner as in Example 1 except that the particle 1 serving as the particle of the cyclic conjugated compound in which the pyrrole rings are bonded by conjugated bonds is changed to a titanyl phthalocyanine (TiOPc) particle.

### (Example 7)

A photoelectric conversion element is obtained in the same manner as in Example 1 except that the particle 1 serving as the particle of the cyclic conjugated compound in which the pyrrole rings are bonded by conjugated bonds is changed to a zinc phthalocyanine (ZnPc) particle.

### (Example 8)

A photoelectric conversion element is obtained in the same manner as in Example 1 except that the particle 1 serving as the particle of the cyclic conjugated compound in which the pyrrole rings are bonded by conjugated bonds is changed to a silicon phthalocyanine dichloride (SiPcCl₂) particle.

### (Example 9)

A photoelectric conversion element is obtained in the same manner as in Example 1 except that the particle 1 serving as the particle of the cyclic conjugated compound in which the of pyrrole rings are bonded by conjugated bonds is changed to a tetraphenylporphyrin (TPP) particle.

### (Example 10)

A photoelectric conversion element is obtained in the same manner as in Example 1 except that 0.07 g of the resin solution 1 is used for the preparation of the charge-transporting layer solution.

### (Example 11)

A photoelectric conversion element is obtained in the same manner as in Example 1 except that 0.4 g of the resin solution 1 is used for the preparation of the charge-transporting layer solution.

### (Example 12)

A photoelectric conversion element is obtained in the same manner as in Example 1 except that 0.005 g of the calixarene compounds are used for the preparation of the charge-transporting layer solution.

### (Example 13)

A photoelectric conversion element is obtained in the same manner as in Example 1 except that 0.05 g of the calixarene compounds are used for the preparation of the charge-transporting layer solution.

### (Example 14)

A photoelectric conversion element is obtained in the same manner as in Example 1 except that 0.06 g of the resin solution 1 is used for the preparation of the charge-transporting layer solution.

### (Example 15)

A photoelectric conversion element is obtained in the same manner as in Example 1 except that 1.0 g of the resin solution 1 is used for the preparation of the charge-transporting layer solution.

### (Example 16)

A photoelectric conversion element is obtained in the same manner as in Example 1 except that 0.004 g of the calixarene compounds are used for the preparation of the charge-transporting layer solution.

### (Example 17)

A photoelectric conversion element is obtained in the same manner as in Example 1 except that 0.1 g of the calixarene compounds are used for the preparation of the charge-transporting layer solution.

### (Example 18)

A photoelectric conversion element is obtained in the same manner as in Example 1 except that the polyvinyl butyral is changed to polyvinyl butyral (product name: BX-1, manufactured by Sekisui Chemical Co., Ltd., glass transition temperature: 95°C).

### (Example 19)

A photoelectric conversion element is obtained in the same manner as in Example 1 except that the polyvinyl butyral is changed to polyvinyl acetal (product name: KS-1, manufactured by Sekisui Chemical Co., Ltd., glass transition temperature: 107°C).

### (Example 20)

A photoelectric conversion element is obtained in the same manner as in Example 1 except that the polyvinyl butyral is changed to polymethyl methacrylate (PMMA, manufactured by Sigma-Aldrich Co. LLC, glass transition temperature: 70°C).

### (Example 21)

A photoelectric conversion element is obtained in the same manner as in Example 1 except that a mixture of compounds represented by the following formulae [C-5] and [C-6] at a mass ratio of 1:1 is used as the calixarene compounds.

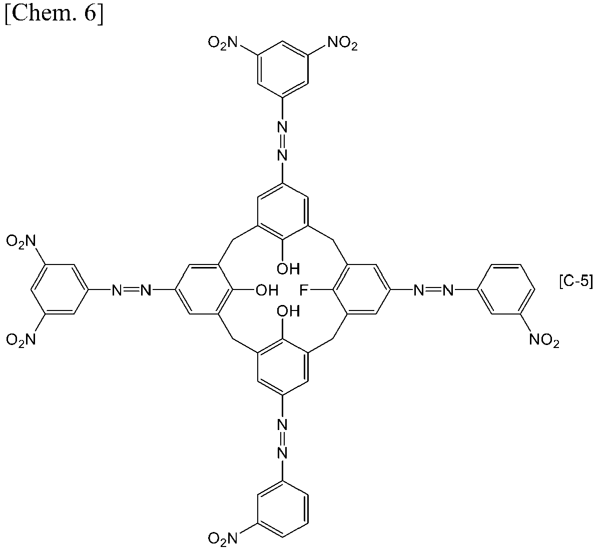

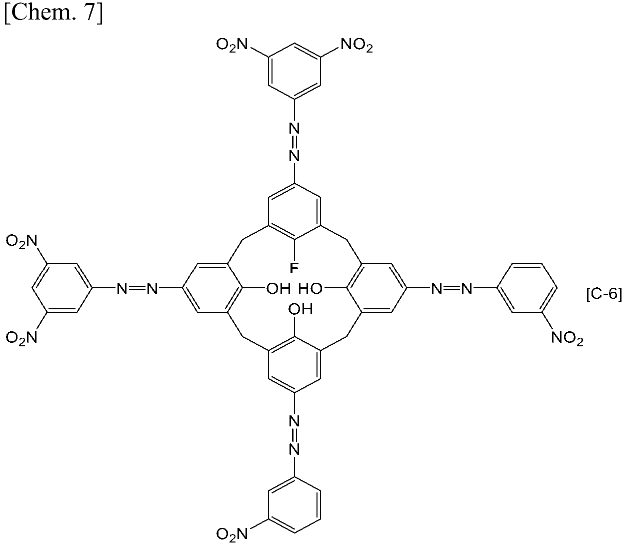

### (Example 22)

A photoelectric conversion element is obtained in the same manner as in Example 1 except that a boron-doped diamond electrode formed by a plasma CVD method is used as the carbon electrode.

### (Comparative Example 1)

A photoelectric conversion element was obtained in the same manner as in Example 1 except that, in the formation of the charge-transporting layer, the calixarene compounds and the resin solution 1 were not used.

### (Comparative Example 2)

A photoelectric conversion element is obtained in the same manner as in Example 1 except that, in the formation of the charge-transporting layer, the calixarene compounds are not used.

### (Comparative Example 3)

A photoelectric conversion element is obtained in the same manner as in Example 1 except that, in the formation of the charge-transporting layer, the resin solution 1 is not used.

### (Comparative Example 4)

A photoelectric conversion element is obtained in the same manner as in Example 1 except that, in the formation of the charge-transporting layer, the calixarene compounds are changed to phenol.

### (Comparative Example 5)

A photoelectric conversion element is obtained in the same manner as in Example 1 except that the polyvinyl butyral is changed to poly(3-hexylthiophene-2,5-diyl) (P3HT, manufactured by Sigma-Aldrich Co. LLC) in the preparation of the resin solution 1.

### (Comparative Example 6)

A photoelectric conversion element is obtained in the same manner as in Example 1 except that, in the formation of the charge-transporting layer, a charge-transporting layer solution 2, which is obtained by dissolving 79.91 mg of Spiro-OMeTAD serving as a charge-transporting material, 34 µL of t-butylpyridine, 10 mg of silver bis(trifluoromethylsulfonyl)imide, and 0.9 mg of a norbornene resin (product name: TOPAS 6013, manufactured by Polyplastics Co., Ltd., glass transition temperature: 130°C) serving as an insulating resin into 1 mL of chlorobenzene, is used.

### [Evaluation]

A power supply (236 model, manufactured by Keithley Instruments, LLC) is connected between the electrodes of the photoelectric conversion element produced in Example 1, and its photoelectric conversion efficiency is measured by: irradiating the element with constant light through use of a solar simulator (manufactured by Yamashita Denso Corporation) having an intensity of 114 mW/cm²; and measuring the generated current and voltage. In addition, each of the ten electrodes for each photoelectric conversion element is subjected to the measurement, and the average of the measured values is adopted as the representative value of the photoelectric conversion element. The results are shown in Table 2.

In Table 2, the photoelectric conversion efficiency in Example 1 is set to 1, and a ratio thereto is shown as the conversion efficiency of each photoelectric conversion element.

### [Table 2]

**Table 2**

| Example | Charge-transportine layer | | | | | | Thin film layer | Carbon electrode | Relative value of conversion efficiency to Example 1 |
|---|---|---|---|---|---|---|---|---|---|
| | Cyclic conjugated compound | Resin in charge-transporting layer | Glass transition temperature of resin [°C] | Content of cyclic conjugated compound relative to resin [times] | Calixarene compound | Content of cyclic conjugated compound relative to calixarene compound [times] | | | |
| Example 1 | OHGaPc | BM-2 | 71°C | 10 | C-1~4 | 10 | | Carbon paste | 1 |
| Example 2 | OHGaPc | BL-5Z | 67°C | 10 | C-1~4 | 10 | | Carbon paste | 0.99 |
| Example 3 | OHGaPc | BM-2 | 71°C | 10 | C-1~4 | 10 | PEAI | Carbon paste | 0.96 |
| Example 4 | ClGaPc | BM-2 | 71°C | 10 | C-1~4 | 10 | | Carbon paste | 0.95 |
| Example 5 | CuPc | BM-2 | 71°C | 10 | C-1~4 | 10 | | Carbon paste | 0.92 |
| Example 6 | TiOPc | BM-2 | 71°C | 10 | C-1~4 | 10 | | Carbon paste | 0.91 |
| Example 7 | ZnPc | BM-2 | 71°C | 10 | C-1~4 | 10 | | Carbon paste | 0.89 |
| Example 8 | SiPcCl₂ | BM-2 | 71°C | 10 | C-1~4 | 10 | | Carbon paste | 0.87 |
| Example 9 | TPP | BM-2 | 71°C | 10 | C-1~4 | 10 | | Carbon paste | 0.83 |
| Example 10 | OHGaPc | BM-2 | 71°C | 29 | C-1~4 | 10 | | Carbon paste | 0.97 |
| Example 11 | OHGaPc | BM-2 | 71°C | 5 | C-1~4 | 10 | | Carbon paste | 0.96 |
| Example 12 | OHGaPc | BM-2 | 71°C | 10 | C-1~4 | 20 | | Carbon paste | 0.94 |
| Example 13 | OHGaPc | BM-2 | 71°C | 10 | C-1~4 | 2 | | Carbon paste | 0.9 |
| Example 14 | OHGaPc | BM-2 | 71°C | 33 | C-1~4 | 10 | | Carbon paste | 0.89 |
| Example 15 | OHGaPc | BM-2 | 71°C | 2 | C-1~4 | 10 | | Carbon paste | 0.61 |
| Example 16 | OHGaPc | BM-2 | 71°C | 10 | C-1~4 | 25 | | Carbon paste | 0.88 |
| Example 17 | OHGaPc | BM-2 | 71°C | 10 | C-1~4 | 1 | | Carbon paste | 0.57 |
| Example 18 | OHGaPc | BX-1 | 95°C | 10 | C-1~4 | 10 | | Carbon paste | 0.97 |
| Example 19 | OHGaPc | KS-1 | 107°C | 10 | C-1~4 | 10 | | Carbon paste | 0.82 |
| Example 20 | OHGaPc | PMMA | 70°C | 10 | C-1~4 | 10 | | Carbon paste | 0.91 |
| Example 21 | OHGaPc | BM-2 | 71°C | 10 | C-5,-6 | 10 | | Carbon paste | 0.89 |
| Example 22 | OHGaPc | BM-2 | 71°C | 10 | C-1~4 | 10 | | Diamond | 0.52 |
| Comparative Example 1 | OHGaPc | | | | | | | Carbon paste | 0.28 |
| Comparative Example 2 | OHGaPc | BM-2 | 71°C | 10 | | | | Carbon paste | 0.19 |
| Comparative Example 3 | OHGaPc | | | | C-1~4 | 10 | | Carbon paste | 0.32 |
| Comparative Example 4 | OHGaPc | BM-2 | 71°C | 10 | phenol | | | Carbon paste | 0.21 |
| Comparative Example 5 | OHGaPc | P3HT | 9.3°C | 10 | C-1~4 | 10 | | Carbon paste | 0.38 |
| Comparative Example 6 | | TOPAS 6013 | 130°C | | | | | Carbon paste | 0.16 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-177318 filed on October 9, 2024, and Japanese Patent Application No. 2024-186701 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element further comprises a charge-transporting layer that is arranged between the photoelectric conversion layer and the first electrode, and is brought into contact with the first electrode,
wherein the charge-transporting layer contains a particle of a cyclic conjugated compound in which pyrrole rings are bonded by conjugated bonds, a calixarene compound, and an insulating resin, and
wherein the first electrode is a carbon electrode.

2. The photoelectric conversion element according to claim 1, wherein the cyclic conjugated compound is a phthalocyanine compound.

3. The photoelectric conversion element according to claim 2, wherein the phthalocyanine compound is a metal phthalocyanine compound having a metal element in a center thereof.

4. The photoelectric conversion element according to claim 3, wherein the metal phthalocyanine compound is a gallium phthalocyanine compound.

5. The photoelectric conversion element according to claim 4, wherein the gallium phthalocyanine compound is a hydroxygallium phthalocyanine compound.

6. The photoelectric conversion element according to any one of claims 1 to 5, wherein the calixarene compound is represented by the following formula [A]: 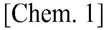 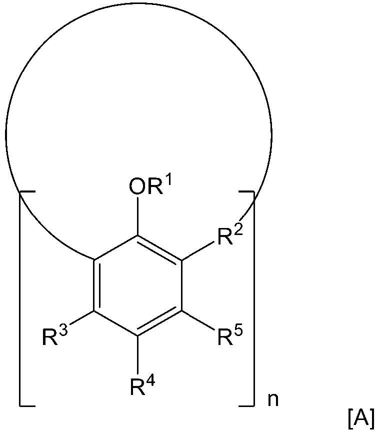 in the formula [A], R¹ to R⁵ are as follows each independently in each repeating unit and each independently for "n" repeating units: R¹ represents a hydrogen atom or an alkyl group; R² represents a substituted or unsubstituted alkylene group; and R³ to R⁵ each represent a hydrogen atom, a substituted or unsubstituted alkyl group, a substituted or unsubstituted -Y-Ar group, a substituted or unsubstituted aromatic hydrocarbon group, or a substituted or unsubstituted heterocyclic group, and at least one of R³ to R⁵ represents a substituted or unsubstituted -Y-Ar group, -Y- of the -Y-Ar group represents -CH=N-, -CH=CH-, or -N=N-, and Ar thereof represents a substituted or unsubstituted aromatic hydrocarbon group, or a substituted or unsubstituted heterocyclic group, and "n" represents an integer of 3 to 20.

7. The photoelectric conversion element according to claim 6, wherein the calixarene compound represented by the formula [A] includes at least one selected from the group consisting of compounds represented by the following formulae [C-1], [C-2], [C-3], and [C-4].

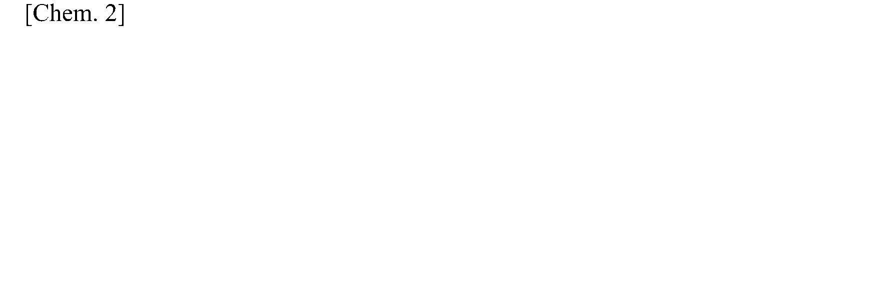

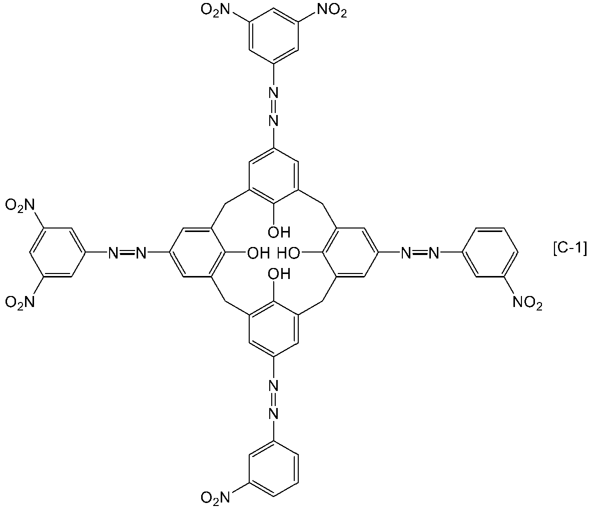

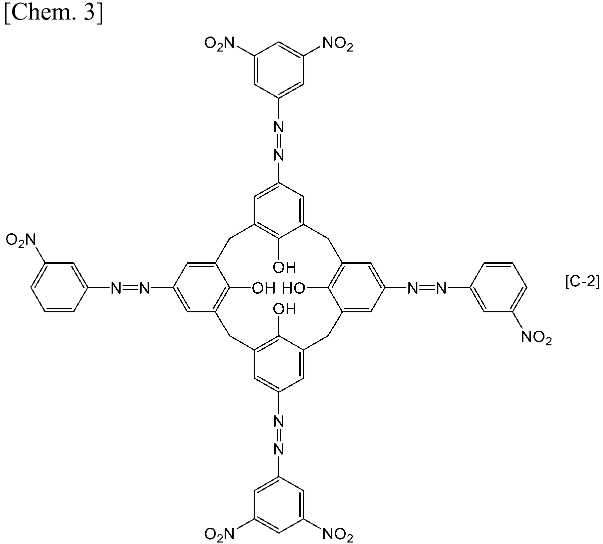

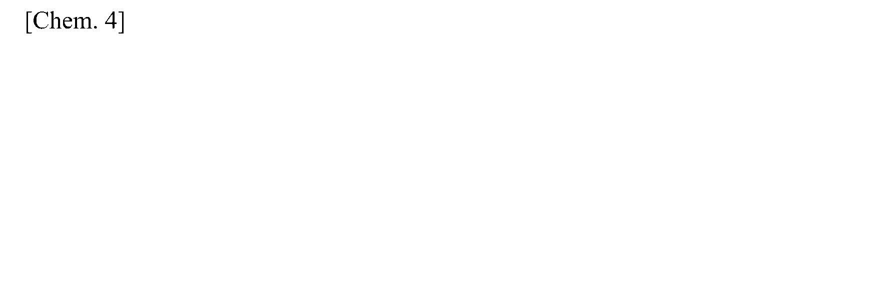

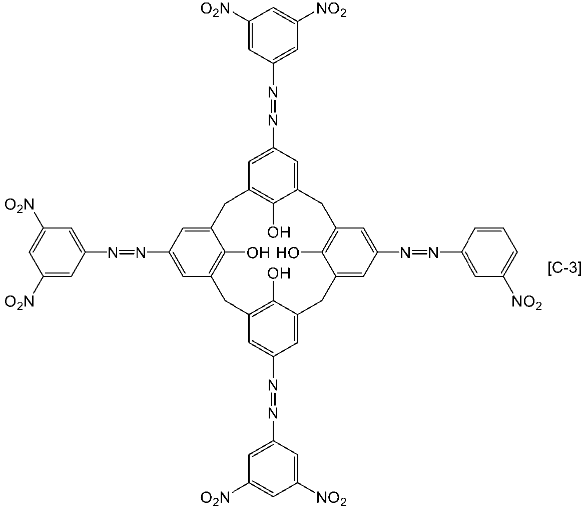

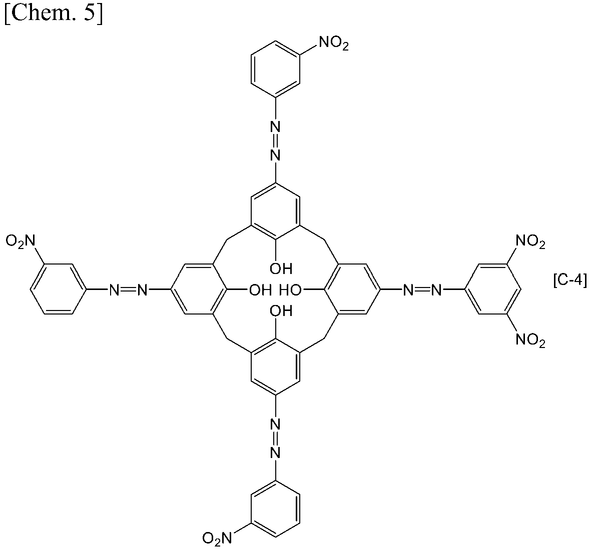

8. The photoelectric conversion element according to any one of claims 1 to 7, wherein the insulating resin has a glass transition temperature of 95°C or less.

9. The photoelectric conversion element according to any one of claims 1 to 8, wherein the insulating resin is a polyvinyl acetal resin or a polyvinyl butyral resin.

10. The photoelectric conversion element according to any one of claims 1 to 9, wherein a content mass of the particle of the cyclic conjugated compound in the charge-transporting layer is 5 to 30 times with respect to a content mass of the insulating resin in the charge-transporting layer.

11. The photoelectric conversion element according to any one of claims 1 to 10, wherein a content mass of the particle of the cyclic conjugated compound in the charge-transporting layer is 2 to 20 times with respect to a content mass of the calixarene compound in the charge-transporting layer.

12. The photoelectric conversion element according to any one of claims 1 to 11, wherein the carbon electrode contains at least one selected from the group consisting of: carbon black; graphite; and a carbon nanotube.

13. A photoelectric conversion apparatus comprising the photoelectric conversion element of any one of claims 1 to 12.

14. A method of producing a photoelectric conversion element including a photoelectric conversion layer, a charge-transporting layer, and an electrode, the method comprising, in this order, the steps of:
forming the photoelectric conversion layer containing a crystal having a perovskite structure;
forming the charge-transporting layer containing a particle of a cyclic conjugated compound in which pyrrole rings are bonded by conjugated bonds, a calixarene compound, and an insulating resin; and
forming the electrode by applying a coating liquid.
